# EUROPEAN PATENT APPLICATION

(11) **EP 1 829 580 A1**
(43) Date of publication of application: **05.09.2007**
(21) Application number: 07250890.6
(22) Date of filing: 02.03.2007
(51) Int. Cl.: A61N 1/24, A61N 1/32, A61F 7/02, A61F 7/08

(54) **Therapy device**

(30) Priority: 04.03.2006 GB 0604404
(71) Applicant: The Dezac Group Limited, Cheltenham Gloucestershire GL50 1SS (GB)
(72) Inventor: Brown, Stephen Colin, Ashton Under Hill Worcestershire WR11 7RF (GB)
(74) Representative: Newell, William Joseph

(57) **Abstract**

A therapy device in the form of a support belt (10) is disclosed which includes heating apparatus (18) in combination with electrical current body stimulation apparatus (22), providing heating and electrical body stimulation during therapy. The device includes a control unit (24) for selectively controlling the amount and/or duration of the therapy provided. A user can create a preferred therapy regime using the control unit. The control unit has means for detecting the correct attachment of the therapy device to the body of a user.

## Description

This invention relates to a therapy device particularly but not exclusively to a multi-function therapy device incorporating heating apparatus and electrical current stimulating apparatus.

Apparatus for promoting localised heat are known, IL0036731A0: Electrically Heated Sauna Belt for Health and Reducing Process (1971) by Rubin A describes an apparatus wom about the body for provision of localised heat by an electrical heating element.

The health benefits of 'sauna' type heating of the body are documented. The increased temperature dilates cutaneous capillary vessels and, to maintain sufficient blood pressure, the cardiac output increases up to three fold. Considerable redistribution of cardiac output takes place. Normally, the skin blood flow is 5-10% of the cardiac output, but can increase to 50-70%. Correspondingly, the blood flow of inner organs and muscles decreases. The changes of blood pressure are moderate. Most often a small decrease of systolic and diastolic blood pressure takes place. It has been shown that the increase of cardiac load in a traditional sauna bath is similar to that seen during brisk walking.

A sauna treatment affects the endocrine system in many ways. According to most studies sauna treatment stimulates the productions of noradrenaline, prolactin, growth hormone and cardiac natriuretic peptide, and activates renninangiotensin- aldosterone system and these hormonal changes can result in an elevated mood.

Furthermore, apparatus for the provision of electrical stimulation, such as Abdominal Muscle Stimulation Belt (GB2369998A) by BMR, are known and have been used as an aid to exercise and muscle tone.

EMS (Electrical Muscle Stimulation) uses electrical current to stimulate the muscles via a pair of electrodes placed upon the skin about a muscle group. The electrical impulse causes the muscles to respond by contracting and relaxing rhythmically. When a muscle contracts as a result of the electrical signal, the chemical changes taking place within the muscles are similar to those associated with voluntary contraction in 'normal exercising'. Over a period of time these contractions will enable an individual to tone and strengthen the muscle.

This invention has come about as a result of the inventor's realisation that the combination of heating and electrical body stimulation has a synergistic effect which is more beneficial to the user than the two therapies applied separately. It is thought that muscles which are subjected to electrical stimulation take less time to tone and strengthen when localised heat (and consequently blood supply) are increased. The efficiency of the electrical stimulating apparatus is improved when perspiration caused by localised heating is present.

According to the present invention there is a provided a therapy device characterised in that the device comprises heating apparatus in combination with electrical current body stimulation apparatus, the device in use being capable of providing heating and electrical body stimulation during therapy.

The present invention is further distinguished by its preferred features which solve further technical problems.

The heating apparatus may comprise one or more of the following: an electrical resistance heater; an exothermic chemical reaction, including the burning of a fuel source; or a preheated mass e.g. water.

The electrical current body stimulation apparatus may comprise one or more of the following: electrical muscle-contracting stimulation (EMS); a transcutaneous electrical nerve stimulator (TENS) or an electro-acupuncture stimulator.

Preferably the therapy device is in the form of apparel (e.g, a belt) wearable by a user.

Preferably the apparel includes support members for providing support to a moveable part of the user's body during use.

Preferably the device or apparel includes a control unit for selectively controlling the amount and/or duration of the therapy provided. The control unit may provide also a therapy safety threshold which cannot be overriden. Preferably the control unit allows the user to select or create a preferred therapy regime.

Preferably the control unit has means for detecting the correct attachment of the therapy device to the body of a user. Preferably the means for detecting comprises two or more electrodes and a circuit for determining the impedance between the said electrodes,

Preferably the said heating apparatus and the said electrical apparatus are separable from the device or apparel.

Preferably the apparel is formed from an elastomeric insulating material such as neoprene. Preferably the apparel contains at least one rigid or semi rigid portion providing support for the joints of a user. Preferably the said portions are removable from the apparel.

Preferably the apparel includes pockets suitable for the introduction of permanent magnets adjacent the body of the user.

The invention extends to an novel combination of features set out above or described below.

A specific embodiment of the invention will now be described by way of example, with reference to the drawings, wherein:
Figure 1 shows a therapy belt according to the invention; and
Figure 2 shows a schematic representation of an electrical circuit used in the embodiment shown in Figure 1.

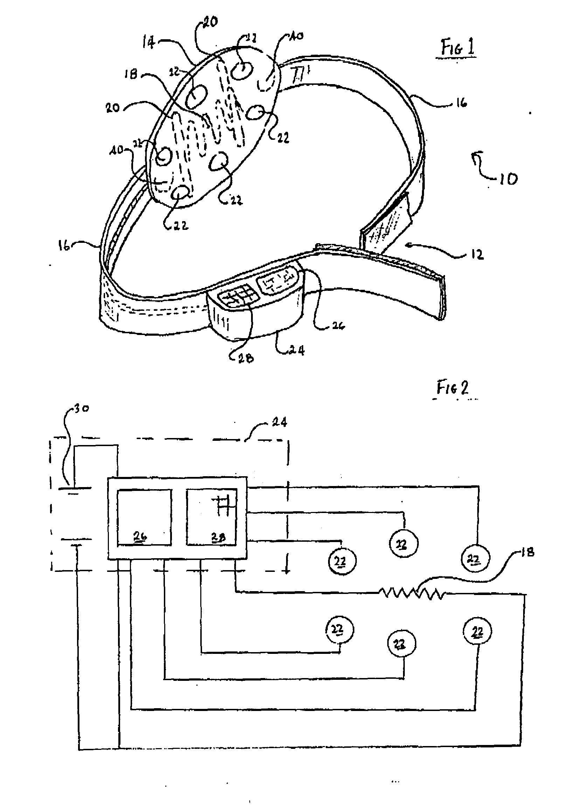

Figure 1 shows a therapy device in the form of a therapy belt 10. The belt 10 is wearable by a user by means of wrapping around a part of the body of a user and securing the belt by means of a hook and loop type fastener buckle 12. The belt 10 has a webbing strap section 16 and a therapy section 14. The therapy section 14 comprises a neoprene cover enclosing a heating element 18 and joint supports 20. On the surface of the neoprene cover are a plurality of electrodes 22. The belt also includes a control unit 24 having a display 26 and a key pad 28.

During use a user wears a belt 10 so that the electrodes 22 come into contact with the user's skin. The electrodes 22 are placed adjacent muscles which require therapy. Heating elements 18 provide localised heating at the treatment area. Supports 20 provide support for the region of the body requiring therapy. During use the user can operate the device using control unit 24 by selecting the appropriate keys on key pad 28 and monitor the progress of the therapy using display 26.

Figure 2 shows a schematic representation of an electrical circuit used in the embodiment shown in Figure 1. The electrical circuit is powered by a rechargeable battery pack 30 which supplies power to the control unit 24. The control unit 24 includes a micro-controller which controls the operation of the device. In particular the micro-controller operates the display 26 and has inputs from key pad 28. The micro-controller also controls the amount and duration of power supply to heating element 18 and the amount and duration of power to electrodes 22.

In use the micro controller of the control unit 24 is pre-programmed with a selection of therapy regimes, and user defined therapy regimes may also be created. The therapy regime may include heating and electrical simulation simultaneously or one after the other. Alternatively solely heating or solely electrical stimulation may be selected. The control unit has a built in safety feature which prevents overheating of the element 18 and over powering or excessive use of the electrodes 22. Additionally, feedback from the electrodes 22 allows the control unit to determine the impedance between two or more electrodes and thus the control unit is able to detect the correct attachment of the belt to the user. The display 26 is capable of providing user information such as the therapy regime selected, the time remaining for the selected therapy, the actual power supplied through the electrodes and heating element, the temperature of the heating element and the status of battery pack 30.

During use the embodiment illustrated in Figures 1 and 2 provides localised heating to a region of the body such as the back, thigh, waist or knee for the purposes of relaxing and loosening muscular tissue and stimulating blood flow thus aiding the body's healing mechanisms for tissue damage. Furthermore the belt promotes localised fluid loss through sweating and allowing the belt to be used cosmetically as part of a slimming regime if required. Additionally, the belt provides electrical stimulation for promoting muscle toning (using EMS), pain reduction (TENS) or other electrical therapy. In this embodiment using these treatments together increases their effectiveness when compared to the same separate treatments. A further function of the belt is to provide support means for helping the wearer with muscle sprain or the like and aiding simple activities, thus limiting chances for affects of further damage while rehabilitating.

One embodiment of the invention is described above and illustrated, however, it will be apparent to the skilled addressee that variants, alternatives, and different techniques are possible within the ambit of the invention.

### Particularly:

The heating element 18 is shown as a electrical resistance. It will be apparent that any other heating apparatus could be used to equal effect e.g. an exothermic chemical reaction for example the burning of a fuel source, or the use of a preheated mass such as water or natural stone.

The electrodes 22 shown are intended to be used with electrical current muscle stimulation (EMS) or transcutaneous electrical nerve stimulation (TENS). However, any other electrical stimulation therapy could be used, for example electro-acupuncture.

The device shown in Figure 1 is a belt. However, it will be apparent that other arrangements for the device could be used. For example a simple pad incorporating the features of the invention could be used or smaller arm or leg mountable apparel could be used also.

The control unit 24 is intended to control both the heating and the electrical stimulation therapies provided. However, separate controls for heating and electrical stimulation may be provided. A visual display unit 26 is illustrated. However, audible or tactile indicators may be provided as an alternative or in addition to the display 26.

The belt is powered using rechargeable battery pack 30. However an external electricity supply may be used in addition to or as an alternative to the battery pack.

The therapy section 14 is separable from the strap 16 to aid cleaning and maintenance. However, particularly for smaller devices, a belt or strap may be made integral with the heating and electrical stimulation parts of the device to form a single piece therapy device, with or without a control unit, and if a control unit is used it need not have a display or key pad. For simple devices an on/off switch would suffice.

The therapy section 14 is described having a neoprene cover although any flexible or semi flexible material could be used in the construction of the pad for example a polymeric material such as rubber or plastics.

Supports 20 may be removable to allow for adjustment and maintenance of the device.

Pockets 40 or other suitable holders for permanent magnets may be provided in the device to improve blood flow.

## Claims

1. A therapy device (10) **characterised in that** the device comprises heating apparatus (18) in combination with electrical current body stimulation apparatus (22), the device in use being capable of providing heating and electrical body stimulation during therapy.

2. A therapy device as claimed in claim 1 wherein the heating apparatus comprises one or more of: an electrical resistance heater; an exothermic chemical reaction, including the burning of a fuel source; or a preheated mass.

3. A therapy device as claimed in claim 1 or 2 wherein the electrical current body stimulation apparatus comprises one or more of: electrical muscle-contracting stimulation (EMS); a transcutaneous electrical nerve stimulator (TENS) or an electro-acupuncture stimulator.

4. A therapy device as claimed in claim 1,2 or 3 wherein the therapy device is in the form of apparel (10) wearable by a user.

5. A therapy device as claimed- in claim 4 wherein the apparel is in the form of a belt (10).

6. A therapy device as claimed in claim 4 or 5 wherein the apparel includes one or more support members (20) for providing support to a moveable part of the user's body during use.

7. A therapy device as claimed in claim 6 wherein the or each support member includes at least one rigid or semi rigid portion (20) providing support for the joints of a user.

8. A therapy device as claimed in claim 7 wherein said portions are removable from the apparel.

9. A therapy device as claimed in claim any one of claims 4, to 8 wherein the apparel includes an elastomeric insulating material such as neoprene.

10. A therapy device as claimed in claim any one of claims 4 to 9 wherein the apparel includes pockets (40).

11. A therapy device as claimed in claim any one preceding claim including a control unit (24) for selectively controlling the amount and/or duration of the therapy provided.

12. A therapy device as claimed in claim 11 wherein the control unit provides also a therapy safety threshold which cannot be overridden.

13. A therapy device as claimed in claim 11 or 12 wherein the control unit allows the user to select or create a preferred therapy regime.

14. A therapy device as claimed in claim 11, 12 or 13 wherein the control unit has means (22) for detecting the correct attachment of the therapy device to the body of a user.

15. A therapy device as claimed in claim 14 wherein the means for detecting comprises two or more electrodes and a circuit for determining the impedance between the said electrodes.

16. A therapy device as claimed in any preceding claim wherein said heating apparatus and said electrical stimulation apparatus are separable from the device or apparel.

17. A therapy device as claimed in any preceding claim including permanent magnets adjacent the body of the user.
